# EUROPÄISCHE PATENTSCHRIFT

(11) **EP 3 397 119 B1**
(45) Veröffentlichungstag und Bekanntmachung des Hinweises auf die Patenterteilung: **23.11.2022**
(21) Anmeldenummer: 16820313.1
(22) Anmeldetag: 30.12.2016
(51) Int. Cl.: A47C 20/04, A47C 31/00, A61B 5/00, A47C 21/00, A47C 27/00, A61B 5/0205, A61B 5/113

(54) **SCHLAF- ODER RUHEMÖBEL MIT EINEM SENSOR**
PIECE OF FURNITURE FOR SLEEPING OR RESTING, HAVING A SENSOR
MEUBLE DE COUCHAGE OU DE REPOS POURVU D'UN CAPTEUR

(30) Priorität: 30.12.2015 DE 202015107148 U; 24.05.2016 DE 102016109524; 06.07.2016 DE 202016103605 U; 07.10.2016 DE 202016105635 U
(43) Veröffentlichungstag der Anmeldung: 07.11.2018
(73) Patentinhaber: DewertOkin Technology Group Co., Ltd., Jiaxing City, Zhejiang Province (CN)
(72) Erfinder: GEHRKE, Karsten, 32457 Porta Westfalica (DE); HILLE, Armin, 33659 Bielefeld (DE); LOLEY, Steffen, 49082 Osnabrück (DE); TEWS, Alexander, 33607 Bielefeld (DE)
(74) Vertreter: Kleine, Hubertus
(86) Internationale Anmeldenummer: PCT/EP2016/082921
(87) Internationale Veröffentlichungsnummer: WO 2017/114950

(56) Entgegenhaltungen:
- WO-A1-2013/173640
- JP-A- 2004 344 675
- JP-A- 2005 177 471
- JP-A- 2005 253 957
- US-A1- 2008 005 838
- US-A1- 2010 101 022
- US-A1- 2012 253 142

## Beschreibung

Die Erfindung betrifft ein Schlaf- oder Ruhemöbel mit einem plattenförmigen Stützelement zur Auflage eines Polsters oder Matratze und mindestens einem Sensor zur Erfassung von Vibrationen, Bewegung und/oder Schall gemäß dem Oberbegriff von Anspruch 1.

Im klinischen Bereich sind Überwachungsgeräte bekannt, die Atmung und/oder Herztätigkeit eines Patienten im Schlaf überwachen, um bei bedenklichen Herzfunktions- und Kreislaufparametern eingreifen zu können.

Inzwischen sind Vorrichtungen zur Überwachung des Schlafzustands anhand von physiologischen Parametern auch für nichtklinische Zwecke im Handel erhältlich. Diese Geräte, die beispielsweise auf einen Nachtisch gestellt werden, erfassen Geräusche und/oder Bewegungszustände während des Schlafs mithilfe von Mikrofonen und/oder Kameras. Aus den erfassten Informationen wird ein Schlafzustand abgeleitet, dessen zeitlicher Verlauf aufgezeichnet wird. Der aufgezeichnete Schlafverlauf kann nachträglich abgerufen und ausgewertet werden. Er kann Aufschluss darüber geben, wie tief und erholsam der Schlaf gewesen ist.

Neben Systemen, die Kamera und/oder Mikrofon einsetzen, ist ein sensorbasiertes System bekannt, bei dem ein druckempfindlicher Sensorstreifen über die Matratze gelegt wird und bei dem dieser Sensorstreifen mit einem Mobiltelefon (Smartphone) verbunden wird, das die Sensordaten aufzeichnet. Aus den Sensordaten werden unter anderem eine Herzfrequenz und eine Atemfrequenz abgeleitet.

Nachteilig an den genannten nichtklinischen Systemen ist, dass die Zuverlässigkeit der Erkennung stark von der korrekten Positionierung der Überwachungsgeräte auf einen Nachtisch bzw. auf oder an der Matratze abhängt. Die Zuverlässigkeit und auch der Benutzungskomfort dieser Geräte sind dadurch eingeschränkt.

Aus der Druckschrift US 2010/0101022 A1 ist ein Klinikbett bekannt, bei dem ein Sensor zur Erfassung von Vibrationen auf einem Stützelement des Betts unter der Matratze angeordnet ist und insoweit von dem Stützelement entkoppelt ist, dass die Vibrationen trotz der Montage an dem (festen) Stützelement erfolgen kann. So wird ein zuverlässiges Sensorsystem geschaffen, das nicht von einer korrekten Positionierung der Überwachungsgeräte auf einen Nachtisch bzw. auf oder an der Matratze abhängt. Allerdings führt die sich zwischen dem Patienten und dem Sensor befindende Matratze zu einer Signaldämfun.

Weitere relevante Dokumente des Standes der Technik, die zum Verständnis der Erfindung beitragen, werden im Folgenden zitiert: JP2005177471A, JP2005253957A, JP2004344675A, US2012/253142A1, US2008/005838A1, WO2013/173640A1.

Es ist eine Aufgabe der vorliegenden Erfindung, ein Schlaf- oder Ruhemöbel der eingangs genannten Art zu schaffen, bei dem der Sensor zuverlässig zur Überwachung des Schlafverhaltens eines Benutzers verwendbar ist und Signale mit guter Signalstärke erfasst.

Diese Aufgabe wird durch ein Schlaf- oder Ruhemöbel mit den Merkmalen des unabhängigen Anspruchs gelöst. Vorteilhafte Ausgestaltungen und Weiterbildungen sind in den abhängigen Ansprüchen angegeben.

Bei einem erfindungsgemäßen Schlaf- oder Ruhemöbel ist der mindestens eine Sensor an einem Abschnitt des Stützelements angeordnet, der von weiteren Abschnitten des Stützelements und/oder des Schlaf- oder Ruhemöbels entkoppelt ist.

Der an dem Stützelement angeordnete Sensor führt zu einem benutzerfreundlichen und im Hinblick auf eine Fehlbedienung, insbesondere Fehlpositionierung, fehlersicheren System zur Erfassung der physiologischen Parameter. Erfasste physiologische Parameter sind bevorzugt eine Herzfrequenz, eine Atemfrequenz und/oder ein Bewegungszustand der das Schlaf- oder Ruhemöbel benutzenden Person. Durch die - akustische - Entkopplung des Abschnitts, an dem der Sensor angeordnet ist, wird eine hohe Empfangsempfindlichkeit des Sensors erzielt. Der entkoppelte Abschnitt stellt gewissermaßen eine vergrößerte Empfangsmembran und somit eine Art Antenne für den Sensor dar. Entsprechend bildet sich eine große Schwingungsamplitude aus, die zu einem starken Signal des Sensors führt. Zudem verhindert die akustische Entkopplung ein Übertragen von Vibrationen, die nicht von der das Schlaf- oder Ruhemöbel benutzenden Person stammen, in Form von Körperschall über das Gestell des Möbels auf den Sensor. Insbesondere wird so ein störender Einfluss von Trittschall, der über einen Fußboden auf das Möbel übertragen wird, verhindert oder minimiert.

Erfindungsgemäß ist der entkoppelte Abschnitt nur durch einen Teil eines plattenförmigen Teils des Stützelements gebildet, der durch mindestens einen Einschnitt von dem plattenförmigen Teil des Stützelements abgeteilt ist. Das plattenförmige Teil kann dabei ein Mittelteil, ein Rückenteil oder ein Beinteil des Stützelements sein.

Weiter erfindungsgemäß umgeben mehrere, voneinander durch verbleibende Stege getrennte Einschnitte den entkoppelten Abschnitt, wodurch der entkoppelte Abschnitt nur durch die Stege mit dem umgebenden plattenförmigen Teil verbunden ist. Durch die Anzahl, Positionierung und Dimensionierung der Stege können die Schwingungseigenschaften des entkoppelten Abschnitts beeinflusst und so eingestellt werden, dass eine gute Signalstärke des Sensors in solchen Frequenzbereichen erzielt wird, die bei einer Bestimmung von physiologischen Parametern eines Benutzers besonders vorteilhaft sind

Das Schlaf- oder Ruhemöbel weist weiter eine mit dem Sensor verbindbare Auswerteeinheit auf, die zur Verarbeitung und Auswertung der Signale des mindestens einen Sensors und zur Erfassung von physiologischen Parametern einer das Schlaf- oder Ruhemöbel benutzenden Person eingerichtet ist. Die erfassten physiologischen Parameter sind beispielsweise eine Herzfrequenz, eine Atemfrequenz, ein Bewegungsverhalten und/oder ein Schnarchverhalten der Person. Um auch kleine Signale zuverlässig auswerten zu können, weist die Auswerteeinheit vorteilhaft einen Filter, insbesondere einen Tief- oder Bandpassfilter zur Signalverarbeitung auf. Alternativ oder zusätzlich kann auch unmittelbar am Sensor bereits eine erste Signalaufbereitung erfolgen, beispielsweise indem ein Signalverstärker und/oder ein analog und/oder digital arbeitender Signalfilter benachbart zum Sensor angeordnet sind oder in ein Sensorgehäuse integriert sind. Es wird so eine weniger störanfällige Übertragung des Messsignals an die Auswerteeinheit erzielt.

Weiter kann die Auswerteeinheit einen Speicher zum Abspeichern eines Zeitverlaufs der physiologischen Parameter aufweisen. Alternativ oder zusätzlich kann die Auswerteeinheit zu diesem Zweck mit einem externen Speicher verbunden sein. Dabei kann es sich zum einen um eine Cloud handeln, also z.B. einen von einem externen Dienstleister angebotenen Speicherplatz, der dezentral und/oder verteilt von Servern bereitgestellt wird, die über das Internet erreichbar sind. Zum anderen kann es sich auch um eine sogenannte persönliche Cloud handeln, bei der ein Speicherort lokal, z. B. in Form eines NAS (Network Attached Storage) - Speichers bereitgestellt wird, der in einem Intranet erreichbar ist. Schließlich wäre auch ein unmittelbar kabelgebunden an die Auswerteinheit angebundener Massenspeicher in diesem Sinne als eine Cloud zu verstehen. Andere Formen einer kabelgebundenen, oder genauer gesagt einer verdrahtungsgebundenen Cloud umfassen USB-Massenspeichersticks oder Speicherkarten wie SD-Karten. Diese Cloud-bildenden Speicherelemente können an verschiedenen Orten und Komponenten vorgesehen sein, z.B. auch in einem PC (Personal Computer) oder einem Smartphone als Mobilgerät.

Die Auswerteeinheit kann zudem eine Überwachungseinrichtung zum Vergleichen der physiologischen Parameter mit vorgegebenen Grenzwerten aufweisen, um in einem Fall, in dem eine Gesundheitsgefahr für die Person erkannt wird, diese oder eine weitere Person gewarnt werden kann. Zu diesem Zweck weist die Auswerteeinheit bevorzugt eine Übertragungseinheit zur Übertragung der physiologischen Parameter an ein Mobilgerät oder eine sonstige externe Einheit auf. Die Übertragungseinheit ist bevorzugt zur drahtlosen Übertragung der physiologischen Parameter an das Mobilgerät eingerichtet, insbesondere über eine WLAN- oder Bluetooth-Übertragungsstrecke. Wenn die physiologischen Parameter an das Mobilgerät übertragen werden, kann ein Vergleich der physiologischen Parameter mit vorgegebenen Grenzwerten auch im Mobilgerät vorgenommen werden. Auch eine drahtgebundene Ankopplung an externe Einheiten ist denkbar, beispielsweise wenn die externe Einheit ein Personalruf-System in einem Pflegeheim ist.

Alternativ kann die Überwachungseinrichtung selbst extern von der Auswerteeinheit ausgebildet sein und mit der Auswerteeinheit verbunden sein. Eine solche externe Überwachungseinrichtung kann z.B. in einem Mobilgerät ausgebildet sein. Die dafür notwendige Funktionalität kann über ein entsprechendes Programm ("App") bereitgestellt werden. Die externe Überwachungseinrichtung kann auch Teil einer Alarmzentrale sein, beispielsweise in einer Pflegeeinrichtung.

Weiter weist das Schlaf- oder Ruhemöbel einen elektromotorischen Möbelantrieb mit Verstellantrieben zum Verstellen von Möbelteilen auf und es ist eine Steuereinrichtung zur Ansteuerung der Verstellantriebe vorgesehen, wobei die Auswerteeinheit mit der Steuereinrichtung gekoppelt ist oder in die Steuereinrichtung integriert ist. Auf diese Weise können Komponenten der Steuereinrichtung, die bei dem elektromotorischen Möbelantrieb bereits vorhanden sind, auch für die Auswerteeinheit genutzt werden, beispielsweise eine Stromversorgungseinheit, Kommunikationseinrichtungen und/oder ein Gehäuse einschließlich der Anschlussmöglichkeiten. Zudem vereinfacht sich eine Verkabelung des Sensors, wenn die vorhandene Struktur des elektromotorischen Möbelantriebs verwendet wird.

In einer weiteren vorteilhaften Ausgestaltung des Schlaf- oder Ruhemöbels ist die Auswerteeinheit für die Signale des Sensors zusätzlich zur Erfassung und Auswertung von Vibrationen und Bewegung eingerichtet, die bei der Betätigung eines oder mehrerer der Verstellantriebe auftreten. Auf diese Weise kann als positiver Nebennutzen der wenigstens eine Sensor verwendet werden, um eine Fehlfunktion und/oder eine Überlastung und/oder eine Nicht-Belastung eines oder mehrerer der Verstellantriebe im Betrieb zu ermitteln. Die ermittelten Zustände deuten auf bereits eingetretene oder eventuell bevorstehende technische Probleme der Verstellantriebe hin oder auf eine Fehlnutzung.

Da der jeweilige Verstellantrieb mit den Möbelbauteilen mechanisch gekoppelt ist, ist der Sensor in der Lage, selbst die kleinsten Vibrationen und/oder Geräusche des jeweiligen Verstellantriebs erfassen zu können. Alle in diesem Zusammenhang stehenden Signale werden durch die Auswerteeinheit erfasst und mittels geeigneter Filter, beispielsweise geeigneter Bandpassfilter, als Signale der Verstellantriebe klassifiziert. Die Aussagen über den Verschleiß- und/oder Geräuschzustand des jeweiligen Verstellantriebs werden gespeichert.

In einer weiteren vorteilhaften Ausgestaltung eines Schlaf- oder Ruhemöbels ist die Auswerteeinheit mit einem Schallaufnehmer zur Aufnahme von Luftschall verbunden. Ein derartiger Schallaufnehmer kann z.B. durch ein Kondensatormikrofon gebildet sein. Das Erfassen von Luftschall kann unterstützend eingesetzt werden, um z.B. ein Schnarchen zu detektieren oder ein Schnarchgeräusch einer von mehreren Person zuordnen zu können, die sich im Schlaf- oder Ruhemöbel befinden. Ferner erfolgt die Verbindung zumindest elektrisch. Bevorzugt ist der Schallaufnehmer in der Auswerteeinheit angeordnet. Dazu kann es zweckdienlich sein, wenn dem Schallaufnehmer Zugang zu dem aufzunehmenden Luftschall gewährt wird. Sollte die Auswerteeinheit ein Gehäuse umfassen, weist dieses im Bereich des Schallaufnehmers Öffnungen auf. Ein derartiger Schallaufnehmer ist insbesondere zur Aufnahme eines physiologischen Parameters basierend auf Schnarchgeräuschen ausgebildet. Ein Schlaf- oder Ruhemöbel kann mehrere derartige Schallaufnehmer sowie wenigstens einen Sensor - wie anmeldungsgemäß erläutert - umfassen bzw. aufweisen.

Ferner können weitere Sensoren zur Erfassung von Körperschall vorgesehen sein, welche mit dem Möbel, der Matratze oder mit einem Möbelbauteil verbunden sind. Die Auswerteeinheit ist dann bevorzugt in der Lage, alle Sensorsignale auszuwerten und einen Zusammenhang durch Vergleich zwischen den Sensorsignalen herzustellen. Schnarcht beispielsweise eine von mehreren im Möbel ruhenden Personen und decken sich Signale aus Sensoren zur Aufnahme von Körperschall und Signale aus Sensoren zur Aufnahme von Luftschall, so kann eindeutig eine Zuordnung erfolgen, welche der Person im Bett gerade schnarcht.

Die Erfindung wird nachfolgend anhand von Ausführungsbeispielen mithilfe von Figuren näher erläutert. Es zeigen:
- Fig. 1: ein erstes Ausführungsbeispiel eines Schlafmöbels mit einem elektromotorischen Möbelantrieb in einem schematischen Blockschaltbild;
- Fig. 2: eine Wiedergabe einer zeitlichen Abhängigkeit von Sensordaten;
- Fig. 3: ein zweites Ausführungsbeispiel eines Schlafmöbels mit einem elektromotorischen Möbelantrieb in einer isometrischen Ansicht;
- Fig. 4: das Schlafmöbel gemäß Fig. 3 in einer isometrischen Ansicht von schräg oben;
- Fig. 5 - 7: jeweils ein weiteres Ausführungsbeispiel eines Schlafmöbels mit elektromotorischem Antrieb und einem Sensor in jeweils einer isometrischen Ansicht; und
- Fig. 8, 9: jeweils eine isometrische Darstellung eines Sensors mit einem Sensorgehäuse aus verschiedenen Blickrichtungen.

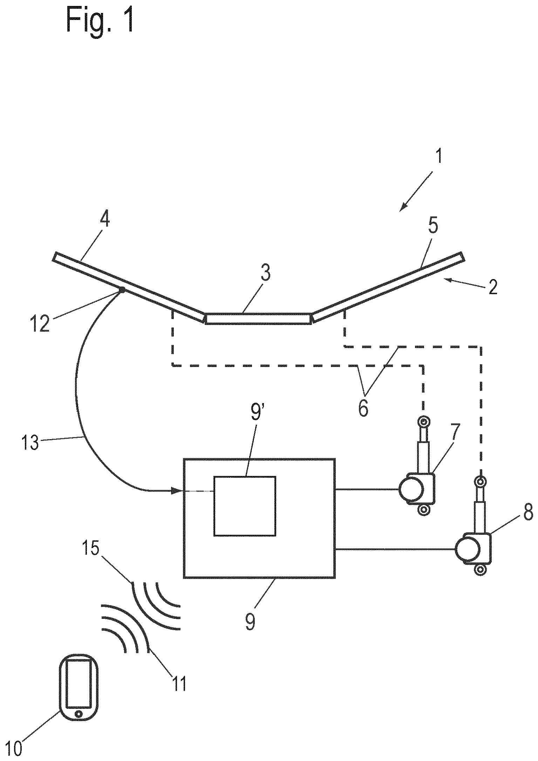

Fig. 1 zeigt ein Bett 1 als Beispiel eines Schlafmöbels in einer schematischen Ansicht.

Das Bett 1 weist wenigstens ein Stützelement 2 zur Aufnahme einer hier nicht dargestellten Matratze auf. Das Bett 1 kann als ein Einzelbett für eine Person oder auch als Doppelbett für mehrere Personen ausgelegt sein. Das Stützelement 2 ist aus mehreren plattenförmigen Teilen ausgebildet und auf ein hier nicht dargestelltes Grundelement, z.B. ein Gestell mit Füßen, aufgelegt oder montiert.

Das Stützelement 2 weist im dargestellten Beispiel ein Rückenteil 4 und ein Beinteil 5 auf, welche relativ zu einem festen Mittelteil 3 oder relativ zu dem Grundelement beweglich gelagert angeordnet sind. Diese bewegliche Anordnung ist beispielsweise mittels eines so genannten hier nicht dargestellten Bewegungsbeschlags realisiert. Die Bewegung ist verschiebbar und/oder schwenkbar ausgebildet.

Das in diesem Beispiel gezeigte Bett 1 ist mit einem elektromotorischen Möbelantrieb ausgestattet. Das beweglich gelagerte Rückenteil 4 und das Beinteil 5 sind dabei jeweils über eine nur schematisch gezeigte Verbindung 6 mit einem elektromotorischen Verstellantrieb 7, 8 gekoppelt. So ist das Rückenteil 4 mit dem elektromotorischen Verstellantrieb 7 gekoppelt. Zur Bewegung bzw. Verstellung des Beinteils 5 ist der elektromotorische Verstellantrieb 8 vorgesehen.

Die elektromotorischen Verstellantriebe 7, 8 sind vorliegend als Linearantriebe ausgebildet. Die Linearantriebe weisen einen oder eine Anzahl Elektromotoren auf, wobei jedem Motor ein Drehzahlreduziergetriebe mit wenigstens einer Getriebestufe nachgeschaltet ist. Dem Drehzahlreduziergetriebe kann ein weiteres Getriebe, beispielsweise in Form eines Gewindespindelgetriebes, nachgeschaltet sein, welches aus der Drehbewegung des Motors eine Linearbewegung eines Abtriebsgliedes erzeugt. Das letzte Getriebeglied oder ein damit verbundenes weiteres Glied bildet das Abtriebsglied. Das Abtriebsglied des jeweiligen elektromotorischen Verstellantriebs steht mit dem jeweiligen Möbelbauteil (Rückenteil 4, Beinteil 5) oder alternativ mit einem mit dem Grundelement verbundenes Bauteil in Verbindung, so dass bei einem Betrieb des Elektromotors des jeweiligen Verstellantriebs 7, 8 die beweglichen Möbelbauteile 4, 5 relativ zueinander bzw. relativ zum Grundelement verstellt werden.

Die elektromotorischen Verstellantriebe 7, 8 sind mit einer Steuereinrichtung 9 verbunden. Diese Verbindung kann z.B. als steckbare Kabelverbindung ausgeführt sein, was hier nicht näher dargestellt ist. Die Steuereinrichtung 9 weist eine elektrische Versorgungseinheit auf, welche die elektrische Energie, z.B. aus einem Stromversorgungsnetz, für die elektromotorischen Verstellantriebe 7, 8 bereitstellt. Dazu ist die Steuereinrichtung 9 über ein in diesem Beispiel nicht gezeigtes Netzkabel mit einem Netzstecker mit einem Netzanschluss verbindbar. Der Netzstecker leitet über das Netzkabel die eingangsseitige Netzspannung zu der elektrischen Versorgungseinheit der Steuereinrichtung 9, welche sekundärseitig eine Kleinspannung in Form einer Gleichspannung abgibt.

Alternativ hierzu ist der Steuereinrichtung 9 eine externe netzabhängige Spannungsversorgung mit Netzeingang und mit sekundärseitigem Kleinspannungsausgang vorgeschaltet, welche über die Leitung die Kleinspannung in Form einer Gleichspannung zuführt.

In einer alternativen Ausgestaltung ist die Steuereinrichtung nicht in einem separaten Gehäuse angeordnet, sondern in einen der Verstellantriebe 7, 8 integriert. Dieser Verstellantrieb stellt dann einen Hauptantrieb dar, an den ggf. weitere Verstellantriebe angeschlossen werden können.

In einer weiteren alternativen Ausgestaltung eines elektromotorischen Möbelantriebs kann die Steuereinrichtung verteilt im System angeordnet sein, derart, dass jeder der Verstellantriebe 7, 8 selbst über eine Motorsteuerung verfügt und eine Bus-Kommunikationsschnittstelle aufweist, über die die Verstellantriebe 7, 8 untereinander und mit weiteren Komponenten verbunden sind. Dabei kann vorgesehen sein, dass wenigsten einer der Verstellantriebe 7, 8 ein eigenes Netzteil zu seiner Stromversorgung oder zur Versorgung mehrerer oder aller vorhandenen Verstellantriebe und/oder ggf. weiterer Systemkomponenten aufweist.

Es ist eine Handbedienung 10 vorgesehen, die Bedienelemente aufweist, mit denen die elektromechanischen Verstellantriebe 7, 8 über die Steuereinrichtung 9 steuerbar sind. Die Handbedienung 10 kann in einem Ausführungsbeispiel über ein Kabel mit der Steuereinrichtung 9 verbunden sein. Alternativ kann die Handbedienung 10 mit einer Übertragungseinrichtung für eine drahtlose Übertragung von Signalen zur Steuereinrichtung 9 versehen sein. Die drahtlose Übertragung kann durch eine Funkübertragungsstrecke, eine optische Übertragungsstrecke (z.B. für Infrarotlicht) und/oder eine Ultraschallübertragungsstrecke realisiert sein, wobei die Steuereinrichtung 9 mit einer jeweiligen entsprechenden Empfangseinheit ausgerüstet ist. Weiter alternativ kann die Handbedienung auch die Steuereinrichtung für die Verstellantriebe bilden, z.B. indem der Betriebsstrom der Verstellantriebe direkt über Schalter der Handbedienung geschaltet wird.

Bei dem dargestellten Ausführungsbeispiel übernimmt ein Mobilgerät 14 die Funktion der Handbedienung 10. Das Mobilgerät 14 kann insbesondere ein handelsübliches Mobiltelefon ("Smartphone") oder ein Tablet-Computer sein. Bevorzugt ist eine Software ("App") für die Funktion als Handbedienung 10 auf dem Mobilgerät 14 installiert. Steueranweisungen an die Verstellantriebe 7, 8 können so über eine drahtlose Übertragungsstrecke 11 von dem als Handbedienung genutzten Mobilgerät 14 an die Steuereinrichtung 9 gesendet werden. Die drahtlose Übertragungsstrecke 11 kann beispielsweise auf einem WLAN (Wireless Lokal Area Network)- oder Bluetooth-Übertragungsweg basieren.

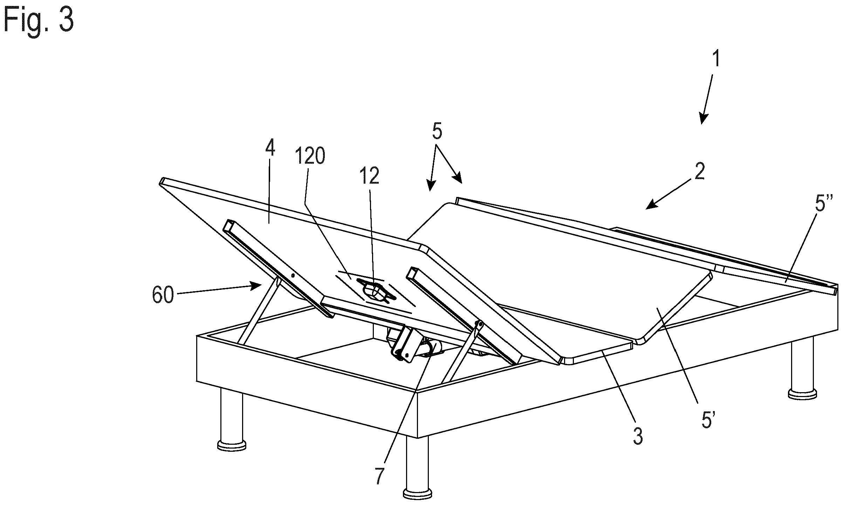

Anmeldungsgemäß ist bei dem dargestellten Bett 1 ein Sensor 12 vorgesehen, der Vibrationen, Bewegung und/oder Schall detektiert. Der Sensor 12 ist im dargestellten Ausführungsbeispiel im Bereich des Rückenteils 4 befestigt. Details zur Anbindung des Sensors 12 an das Bett 1 werden im Zusammenhang mit den Figuren 3 bis 7 näher dargestellt.

Der Sensor 12 ist beispielsweise als piezoelektrisches Bauteil oder als elektromagnetisches Bauteil ausgebildet und ist empfindlich für Schwingungen und Bewegungen der Unterlage, auf der er befestigt ist, vorliegend also für Schwingungen (Vibrationen) bzw. Bewegungen, die der Rahmen des Rückenteils 4 erfährt. Derartige Vibrationen umfassen auch Körperschall, der von einer im Bett 1 ruhenden Person über die Matratze M von dem Rückenteil aufgenommen und weitergeleitet wird. Unter "Bewegungen" sind insbesondere niederfrequente Schwingungen und Auslenkungen des Sensors 12 zu verstehen, deren Frequenz im Hertz- oder Sub-Hertz-Bereich liegt. Ein weiterer für diesen Zweck geeigneter Sensortyp ist ein elektromechanischer Sensor, z.B. ein mikromechanischer Beschleunigungssensor.

Zusätzlich kann der Sensor 12 empfindlich für (Luft-) Schallwellen sein und in diesem Sinne als ein Mikrofon fungieren. Alternativ kann zum Zwecke der Erfassung von Luftschall ein separates Mikrofon, beispielsweise ein Kondensatormikrofon, als zusätzlicher Sensor vorhanden sein.

Der Sensor 12 ist über ein Sensorkabel 13 mit einer Auswerteeinheit 9', die vorliegend in der Steuereinrichtung 9 angeordnet ist, verbunden. Alternativ ist es möglich, die Auswerteeinheit 9' separat von der Steuereinrichtung 9 in einem eigenen Gehäuse auszubilden. Zur Übertragung der ermittelten physiologischen Parameter kann die Auswerteeinheit 9' dann mit der Steuereinrichtung 9 elektrisch gekoppelt sein. Falls benötigt, wird über das Sensorkabel 13 eine Stromversorgung für den Sensor 12 bereitgestellt und es werden vom Sensor 12 ausgegebene Signale an die Auswerteeinheit 9' weitergeleitet. In einer alternativen Ausgestaltung kann der Sensor 12 über eine drahtlose Verbindung, beispielsweise eine Funkverbindung, mit der Auswerteeinheit 9' gekoppelt sein.

In dem Fall ist der Sensor 12 mit einer eigenen Energieversorgung, beispielsweise in Form einer gegebenenfalls wiederaufladbaren Batterie versehen.

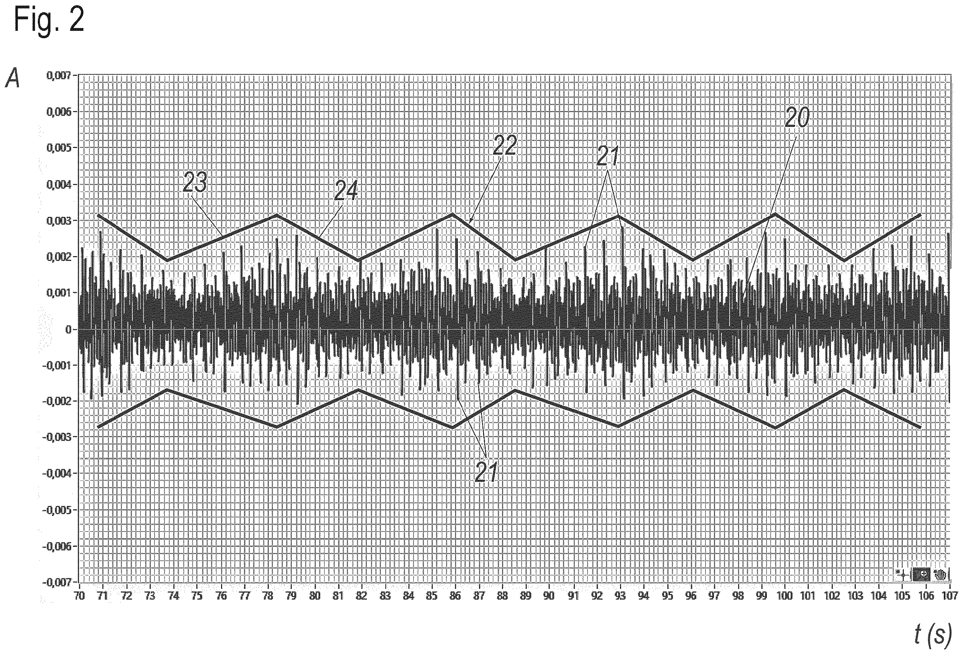

Die Auswerteeinheit 9' umfasst z.B. Verstärker und Filtereinheiten, die es ermöglichen, aus dem vom Sensor 12 übermittelten Signal auf bestimmte Körperfunktionen einer sich im Bett 1 befindenden Person zu schließen. Insbesondere ist die Auswerteeinheit dazu eingerichtet, aus den Signalen des Sensors 12 physiologische Parameter der Person zu ermitteln. Solche Parameter betreffen beispielsweise Herz- und Kreislauffunktionen und umfassen z.B. eine Herzfrequenz und eine Atemfrequenz. Weiter kann ermittelt werden, ob die sich im Bett befindende Person schnarcht. Zudem werden Bewegungen der Person erfasst. Details zur Ermittlung der genannten Parameter aus den Signalen des Sensors 12 werden weiter unten im Zusammenhang mit Fig. 2 näher erläutert.

Die ermittelten Parameter werden entweder unmittelbar oder nach Zwischenspeicherung in der Auswerteeinheit 9' als drahtlose Signale 15 an ein Mobilgerät 14 übertragen. Das Mobilgerät 14 kann insbesondere ein handelsübliches Mobiltelefon ("Smartphone") oder ein Tablet-Computer sein und ist mit einer entsprechenden Software ("App") ausgestattet, die eine Auswertung und bevorzugt grafische Darstellung der Zeitabhängigkeit der ermittelten Schlafparameter ermöglicht. Als Übertragungsweg für die drahtlosen Signale 15 kann beispielsweise WLAN (Wireless Lokal Area Network) oder Bluetooth eingesetzt werden.

Zudem kann in der Auswerteeinheit 9' ein Vergleich der gemessenen physiologischen Parameter mit vorgegebenen Grenzwerten für diese Parameter vorgesehen sein. Werden die ermittelten Parameter unmittelbar, das heißt ohne längere Zwischenspeicherung in der Auswerteeinheit 9', während der Schlafphase an das Mobilgerät 14 übertragen, kann alternativ oder zusätzlich dort ein solcher Vergleich erfolgen. Bei Über- oder Unterschreiten der Grenzwerte bzw. bei einem Verlassen eines oder mehrerer der Parameter aus einem vorgegebenen Bereich ist vorgesehen, dass die Steuereinrichtung 9 bzw. das Mobilgerät 14 ein Alarmsignal ausgibt. Dieses Alarmsignal kann optisch und/oder akustisch unmittelbar von der Auswerteeinheit 9' bzw. der Steuereinrichtung 9 oder dem Mobilgerät 14 ausgegeben werden. Alternativ oder zusätzlich kann vorgesehen sein, dass das Mobilgerät 14 eine Alarmmeldung über eine weitere, hier nicht dargestellte drahtlose Übertragungsstrecke (z.B. WLAN, Mobilfunknetz) abgibt. Auf diese Weise kann eine weitere Person benachrichtigt werden, falls sich ungewöhnliche Schlafparameter zeigen. Das dargestellte Bett 1 bzw. der elektromotorische Möbelantrieb mit dem Sensor 12 kann so auch zur klinischen Überwachung bzw. zur Patientenüberwachung oder zur Überwachung von Kleinkindern zum Schutz vor plötzlichem Kindstod eingesetzt werden.

Bei dem dargestellten Ausführungsbeispiel ist der Sensor 12 als einziger Sensor an dem Rückenteil 4 angeordnet. Weiter ist die Verwendung mehrerer Sensoren 12, die an unterschiedlichen Stellen im oder am Bett 1 positioniert sind, möglich. Dabei können mehrere unterschiedliche Arten (Typen) von Sensoren 12 in dem Bett 1 positioniert sein. Die verschiedenen Sensortypen zeichnen sich durch charakteristische Frequenzbereiche aus, für die sie besonders geeignet sind. Die Kombination verschiedener Sensorarten erlaubt es, ein besonders breites Frequenzspektrum aufnehmen und analysieren zu können.

Der jeweilige Sensor 12 wird dabei an die für ihn ideale Position gebracht, wo das für ihn detektierbare Signal besonders gut ausgeprägt ist. So ist beispielsweise die Position eines körperschallaufnehmenden Sensors 12 in Form eines Piezosensors oder in Form eines Beschleunigungssensors idealerweise der Rückenbereich oder der Mittelbereich eines Bettes 1, während ein Luftschall aufnehmender Sensor 12 beispielsweise in Form eines Mikrofons in der Nähe der oberen Atemwege wie Mund oder Nase einer im Bett 1 ruhenden Person angeordnet ist.

Die Verbindung mit der Auswerteeinheit 9' bzw. der Steuereinrichtung 9, die innerhalb des Betts 1 angeordnet ist, verhindert, dass das Sensorkabel 13 außerhalb des Bettes 1 verlegt werden muss. Die Fixierung des Sensors 12 im oder am Bett 1 stellt eine jederzeit korrekte Positionierung des Sensors 12 und damit eine zuverlässige Auswertung der Daten des Sensors 12 sicher.

Fig. 2 zeigt einen Ausschnitt eines gemessenen Signals 20 des Sensors 12 in einem Diagramm. Auf der horizontalen Achse ist der Zeitverlauf t in Sekunden angegeben. Auf der vertikalen Achse ist eine Signalamplitude A in willkürlichen Einheiten dargestellt.

Der gezeigte Ausschnitt des Signalverlaufs des Signals 20 ist während einer ruhigen Schlafphase ohne Bewegung und ohne Schnarchen der beobachteten Person. Eine Bewegung der Person äußert sich in Amplituden, die die dargestellte um einen Faktor von einigen 10-100 übersteigen. Bewegungen lassen sich daher sehr leicht identifizieren. Auch ein Schnarchen und die damit einhergehenden Vibrationen bzw. Bewegungen sind von dem dargestellten Signalverlauf deutlich unterscheidbar, da sie sich in einer um ein mehrfaches größeren Amplitude widerspiegeln.

In dem in Fig. 2 gezeigten Verlauf des Signals 20 sind regelmäßige Peaks 21 beobachtbar, die vom Herzschlag der Person herrühren und nachfolgend Herzschlag-Peaks 21 genannt werden. Aus dem Abstand der Herzschlag-Peaks 21 kann eine Herzfrequenz ermittelt werden. Der zeitliche Abstand benachbarter Herzschlag-Peaks 21 lässt Aussagen über die Puls-Gleichmäßigkeit zu, die ein Maß für die Tiefe des Schlafes sein kann.

Weiterhin ist in Fig. 2 zu erkennen, dass die Amplitude der Herzschlag-Peaks 21 regelmäßig mit einer geringeren Frequenz variiert. Diese Variation wird durch eine Einhüllende 22 wiedergegeben. Die Einhüllende 22 zeigt sich abwechselnde ansteigende Flanken 23 und abfallende Flanken 24. Der Verlauf der Einhüllenden 22 ist mit der Atmung der Person korreliert. Die ansteigenden Flanken 23 kennzeichnen eine Einatemphase und die absteigende Flanke 24 eine Ausatemphase.

Das Beispiel der Fig. 2 zeigt, wie aus den Signalen des Sensors 12 auf Herz-Kreislaufparameter geschlossen werden kann, vorliegend auf Puls- und Atmung. Auf ähnliche Weise können weitere Schlafparameter, wie Bewegungszustände und ein Schnarchen ermittelt werden.

Zur Auswertung der Signale 20 erfolgt eine Filterung der Rohsignale des Sensors 12, insbesondere mittels eines Tiefpassfilters. Auch die Verwendung eines Bandpassfilters mit geeigneten Eckfrequenzen ist möglich. Tiefpass-bzw. Bandpassfilter dienen der Eliminierung von Störfrequenzen. Bevorzugt erfolgt die Verarbeitung der Signale mit Hilfe eines digitalen Signalprozessors (DSP).

Der Sensor 12 kann zusätzlich oder alternativ auch einer Überwachung der korrekten Funktion des elektromotorischen Antriebs dienen. Eine Betätigung der Verstellantriebe 7, 8 führt zu einer Bewegung der bewegten Möbelteile, beispielsweise des Rückenteils 4 und/oder des Beinteils 5. Zusätzlich resultiert die Betätigung der Verstellantriebe 7, 8 in Vibrationen dieser Möbelteile und auch des gesamten Möbels, die ebenfalls vom Sensor 12 erfasst werden. Diese Vibrationen treten in einem typischen Frequenzbereich auf. Der Signalverlauf spiegelt die Motorbewegung der Verstellantriebe 7, 8 wider. Ein erster typischer relevanter Frequenzbereich liegt im Bereich der Motordrehzahl der Motoren der Verstellantriebe 7, 8. In diesem Frequenzbereich zeigen sich Fehler am Motor selbst oder einem Abtriebszahnrad. Ein weiterer typischer relevanter Frequenzbereich entspricht einem ganzzahligen Bruchteil gemäß einem Übersetzungsverhältnis des Getriebes, das etwa 1:30 bis 1:50 beträgt. In diesem Frequenzbereich deuten sich Fehler in nachgeordneten Getriebestufen oder Wälzlagern an. Ein dritter typischer Frequenzbereich liegt im Bereich von Quietschgeräuschen von Scharnieren, die Teil eines Möbelbeschlags sind. Form und Amplitude sind zum einen typisch für den verwendeten Verstellantrieb 7, 8, zum anderen geben sie Aufschluss über die korrekte Funktion der Verstellantriebe 7, 8 sowie deren Verschleißzustand.

Auch eine Überbelastung einer der Verstellantriebe 7, 8 kann anhand der Signalform der Signale des Sensors 12 erkannt werden. Der Sensor 12 kann somit beispielsweise als Einklemmschutz fungieren, wobei die Steuereinrichtung 9 bei Überbelastung eines der Verstellantriebe 7, 8 diesen Antrieb stoppt bzw. in Gegenrichtung laufen lässt. Auch eine Unterlast am Verstellantrieb 7, 8 kann ein Indiz für ein Einklemmen sein, beispielsweise wenn ein Möbelteil (Rückenteil 4, Beinteil 5) abgelassen wird und der Verstellmotor 7, 8 nahezu kraftlos betrieben wird, deutet dieses auf ein Einklemmen eines Körperteils unter dem sich hinab senkenden bewegten Möbelteil hin. Ein belastungslos betriebener Verstellantrieb 7, 8 ist ebenfalls anhand der Signale des Sensors 12 identifizierbar.

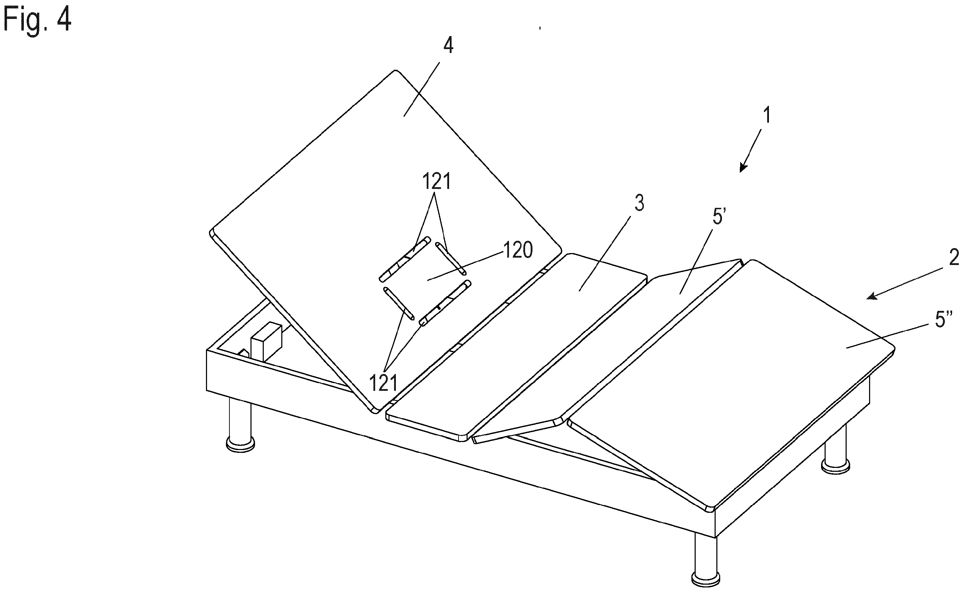

In den Figuren 3 und 4 ist ein weiteres Ausführungsbeispiel eines Betts 1 als Beispiel eines Schlafmöbels mit einem elektromotorischen Möbelantrieb mit Verstellantrieben 7, 8 sowie einem Sensor 12 zur Erfassung von Vibrationen, Bewegungen und/oder Schall dargestellt. In diesem wie allen weiteren Ausführungsbeispielen kennzeichnen gleiche Bezugszeichen gleiche bzw. gleichwirkende Elemente wie bei dem zuvor beschriebenen Beispiel.

Aus Gründen der Übersichtlichkeit ist in den Figuren 3 und 4 eine Steuereinrichtung 9, eine Auswerteeinheit 9', eine Handbedienung 10 mit Übertragungsstrecke 11 sowie ein Mobilgerät 14 und die mit diesem ausgetauschten drahtlosen Signale 15, wie sie in den Figuren 1 gezeigt sind, nicht dargestellt.

In den Figuren 3 und 4 ist das Bett 1 in jeweils einer isometrischen Ansicht aus zwei verschiedenen Blickrichtungen wiedergegeben. Wiederum weist das Bett 1 ein Gestell mit Füßen auf, das ein Grundelement bildet. Es versteht sich, dass anstelle des rahmenartigen Gestells mit den separat ausgebildeten Füßen auch ein eher kastenförmiges Gestell mit vier Seitenwänden, die bis zum Boden reichen, vorgesehen sein kann.

Das Grundelement trägt ein Stützelement 2, das eine Polsterung, insbesondere eine hier nicht dargestellte Matratze aufnimmt. Auch wenn das wiedergegebene Bett 1 ein Einzelbett für eine Person ist, kann es in gleicher Weise als ein Doppelbett für mehrere Personen ausgelegt sein.

Vorliegend weist das Stützelement 2 keinen tragenden Rahmen auf, der z. B. ein Lattenrost als Unterlage der Polsterung bzw. der Matratze aufnimmt, sondern im Wesentlichen selbsttragende Platten. Ein derartiges Bettkonzept wird beispielsweise bei sogenannten Boxspringbetten umgesetzt, bei denen die Matratze dicker als herkömmliche Matratzen ist und Federelemente beinhaltet, die ohne eine federnde Unterlage, wie sie beispielsweise ein Lattenrost darstellt, einen ausreichenden Schlafkomfort bietet.

Das Stützelement 2 weist wiederum mehrere gegeneinander verstellbare Teile auf, konkret ein nicht schwenkbares Mittelteil 3, ein diesem gegenüber schwenkbares Rückenteil 4 und ein hier zweiteiliges Beinteil 5. Ein erster Abschnitt 5' des Beinteils 5 ist dabei schwenkbar gegenüber dem Stützelement 2. Ein zweiter Abschnitt 5" des Beinteils 5 ist an einer freien Querseite des ersten Abschnitts 5' gelenkig mit diesem verbunden und wird entsprechend an der Verbindungslinie der beiden Abschnitte 5', 5" mit angehoben.

Entlang der Verbindungslinien zwischen dem nicht schwenkbaren Mittelteil 3 und dem Rückenteilteil 4 bzw. dem ersten Abschnitt 5' des Beinteils 5, sowie an der Verbindungslinie zwischen dem ersten und dem zweiten Abschnitt 5', 5" des Beinteils 5 sind Schwenkbeschläge, z. B. in Form von Scharnierleisten angeordnet. Am Rückenteil 4 ist zur Kraftübertragung ein u-förmiger Rahmen als Bewegungsbeschlag 60 angeschraubt, dessen Basis in Querrichtung des Betts 1 im Bereich der Verbindung von Rückenteil 4 und Mittelteil 3 verläuft. Der Bewegungsbeschlag 60 übernimmt die Funktion der in Fig. 1 dargestellten Verbindung 6 zwischen dem Verstellantrieb 7 und dem Rückenteil. Der Mittelteil 3 ist vorliegend im festen Abstand zu dem Grundelement.

In Fig. 4 ist das Bett 1 aus Fig. 3 in einer isometrischen Schrägansicht von schräg oben dargestellt. In dieser Figur ist erkennbar, dass im Bereich des hier nicht sichtbaren Sensors 12 vier rechteckförmig angeordnete Einschnitte 121 einen Abschnitt 120 des Rückenteils 4 vom übrigen Bereich des Rückenteils 4 umgeben. Durch die Einschnitte 121 wird dieser Abschnitt 120 akustisch, d.h. im Hinblick auf seine Schwingungsfähigkeit, von dem übrigen Rückenteil 4 entkoppelt. Auf der Rückseite dieses entkoppelten Abschnitts 120 ist der Sensor 12 montiert. Der entkoppelte Abschnitt 120 wird daher nachfolgend auch als Sensorplatte 120 bezeichnet. Zwischen den Einschnitten 121 verbleiben an jeweils den Ecken der Sensorplatte 120 nur relativ schmale Stege.

Die Entkopplung bietet den Vorteil, dass die Sensorplatte 120 gegenüber dem Rückenteil 4 ein schwingungsfähiges System darstellt, das als eine Antenne für Körperschall und Luftschall wirkt und auf den Sensor 12 weiterleitet. Die Sensorplatte 120 stellt gewissermaßen eine vergrößerte Empfangsmembran für den Sensor 12 dar. Entsprechend kann sich an der Sensorplatte 120 eine große Schwingungsamplitude ausbilden, die zu einem starken Signal des Sensors 12 führt. Die Positionierung der Sensorplatte 120 im unteren Rückenbereich ist vorteilhaft, da üblicherweise in diesem Bereich durch Atmung und Herzschlag einer Person hervorgerufene Signale am deutlichsten zu detektieren sind.

Die als Antenne wirkende Sensorplatte 120 verfügt somit über die Eigenschaft, die von der sich im Möbel oder im Bett 1 befindenden Person in Form von Vibrationen bzw. Schall gesendeten physiologischen Signale wie Atmungssignale, Herzsignale, Bewegungssignale, Schnarchsignale zu empfangen und dem wenigstens einen Sensor 12 bereit zu stellen. Die Antenne bzw. die Sensorplatte 120 kann somit wenigstens als Signalaufnahmeeinheit verstanden werden. Sie ist ferner als Signalweiterleitungseinheit ausgebildet. Für eine optimale Aufnahme und/oder Weiterleitung der Signale ist die Sensorplatte 120 aus einem geeigneten Werkstoff gebildet, der eine geringe Dämpfung aufweist. Ferner ist die Geometrie der Sensorplatte 120 gewissermaßen schalloptimiert ausgebildet. Werkstoff und/oder Geometrie sind so gewählt, dass eine Durchleitung sowie eine Weiterleitung von Schall und/oder Vibrationen zu dem jeweiligen Sensor 12 möglichst dämpfungsfrei erfolgt. Als Werkstoffe kommen feste Holzwerkstoffe sowie Holzfaserwerkstoffe zum Einsatz. Der Einsatz von Kunststoffen ist ebenfalls denkbar, wobei eine Faserverstärkung des Werkstoffes vorteilhafte Schalleigenschaften fördert. Ein Einsatz von Metall wie beispielsweise Aluminium ist ebenfalls denkbar.

Eine weitere geometrische Ausgestaltung einer Sensorplatte 120 sieht einen aus einem Kunststoff geformten räumlichen Körper vor. Eine andere geometrische Ausgestaltung einer Sensorplatte 120 sieht einen aus einem Holzfaserwerkstoff geformten ebenen Körper vor.

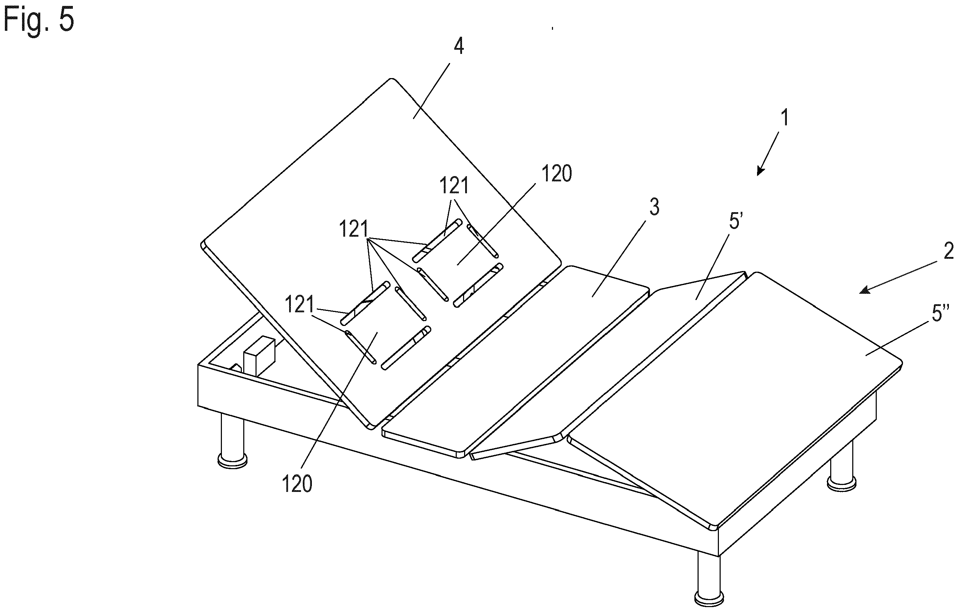

In Figur 5 ist ein weiteres Ausführungsbeispiel eines Bettes 1 als Beispiel für ein Schlaf- und Ruhemöbel in gleicher Art wie in den Figuren 4 und 5 wiedergegeben. Bei diesem Ausführungsbeispiel ist nicht eine Sensorplatte 120 mittig im unteren Drittel des Rückenteils 4 angeordnet, sondern zwei nebeneinander positionierte Sensorplatten 120 sind jeweils in der unteren linken und unteren rechten Hälfte des Rückenteils 4 vorgesehen. Diese Anordnung dient der möglichst unabhängigen Erfassung der Schlafgeräusche und Vibrationen bzw. Bewegungen und damit physiologischen Parametern von zwei nebeneinander im Bett 1 liegender Personen. Beim Ausführungsbeispiel der Fig. 5 sind wiederum durch Einschnitte 121 separierte, aber nicht vollständig getrennte Bereiche des Rückenteils 4 als Sensorplatten 120 ausgebildet. Unter jeder dieser Sensorplatten 120 ist ein eigener Sensor 12 montiert. Die dargestellte Anordnung mit zwei Sensorplatten 120 und zwei Sensoren 12 kann auch bei einem Einzelbett 1 verwendet werden, wobei die Sensorsignale der einzelnen Sensoren 12 entweder gemittelt werden oder das jeweils stärkere Signal eines der Sensoren 12 berücksichtigt wird, je nachdem, wie die Einzelperson im Bett 1 liegt.

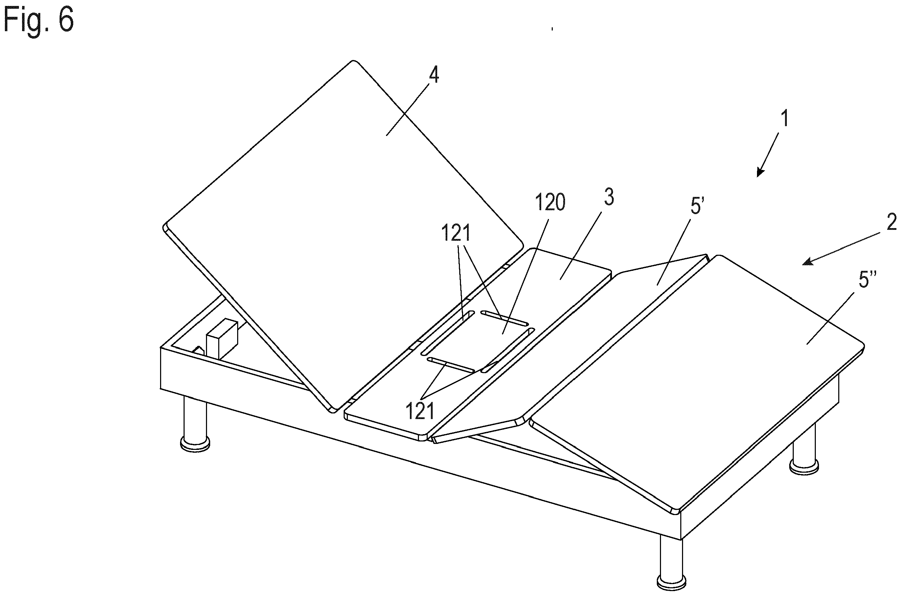

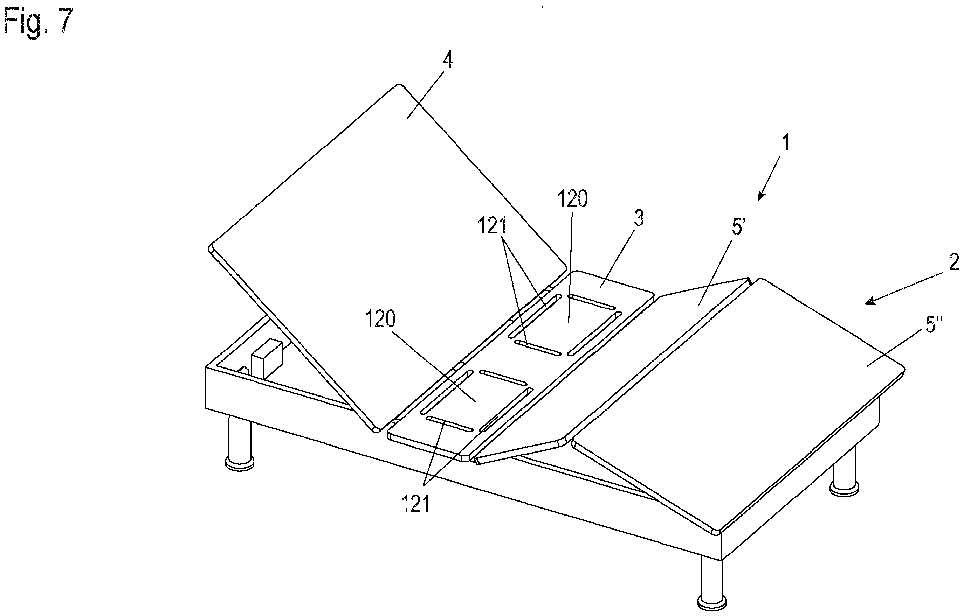

In den Figuren 6 und 7 sind zwei weitere Ausführungsbeispiele eines Bettes 1 als Beispiel eines Schlaf- oder Ruhemöbels gemäß der Erfindung dargestellt.

Bei diesen Ausführungsbeispielen ist die Sensorplatte 120 bzw. sind die Sensorplatten 120 nicht im Rückenteil 4, sondern im Mittelteil 3 angeordnet. In Versuchen hat sich herausgestellt, dass auch im Bereich des Mittelteils 3 in dem der untere Rücken oder das Gesäß von Personen im Schlaf positioniert ist, ebenfalls sehr gut geeignet ist, um Schlafgeräusche, Bewegungen und Vibrationen aufzufangen, die einen Rückschluss auf physiologische Parameter zulassen. Das Mittelteil 3 kann dabei in Bezug auf einen Rahmen des Betts 1 feststehend angeordnet sein. Bei sogenannten "wall hugger"-Betten wird bei einem Verschwenken des Rückenteils 4 das Mittelteil 3 in Richtung des Kopfendes des Bettes 1 verschoben, um die Entstehung eines Spaltes zwischen dem Rückenteil 4 und einer Wand, an der das Bett mit seinem Kopfende steht, zu verhindern. Weiter alternativ ist auch ein leichtes Verschwenken des Mittelteils 3 denkbar.

Ein Vorteil der Anordnung der Sensorplatte 120 oder mehrerer Sensorplatten 120 am Mittelteil 3 liegt darin, dass die Belastung aufgrund der Gewichtskraft der im Bett 1 ruhenden Person(en) unabhängig von der Einstellung des Rückenteils 4 oder des Beinteils 5 im Wesentlichen konstant ist. Die mechanischen Randbedingungen beeinflussen den Sensor 12 bzw. die Entkopplung der Sensorplatte 120. Je konstanter die Randbedingungen sind, umso eindeutiger können die gemessenen Signale des Sensors 12 ausgewertet werden.

In den dargestellten Ausführungsbeispielen ist entweder analog zu der Figur 4 jeweils eine mittige Sensorplatte 120 mit zugeordnetem Sensor 12 vorgesehen, oder jeweils 2 nebeneinander angeordnete in dem Beispiel der Figur 6. Wiederum sind zur Entkopplung der Sensorplatte 120 schlitzartige, nicht miteinander verbundenen Einschnitte 121 eingesetzt.

Bei allen zuvor dargestellten Ausführungsbeispielen ist ein möglichst verlustarmes Übertragen der Vibrationen, insbesondere von Körperschall, von dem entkoppelten Abschnitt 120 bzw. von der Sensorplatte 120 auf den Sensor 12 von Bedeutung. Sensor 12 und der entkoppelte Abschnitt 120 bzw. die Sensorplatte 120 weisen jeweils eine Verbindung zur Übertragung von Schall und/oder Körperschall und/oder Vibrationen auf.

In bevorzugter Weise ist die Verbindung fest. Alternativ ist die Verbindung stoffschlüssig. Ferner lässt die Verbindung bevorzugt keine Relativbewegungen zwischen Sensor 12 und Sensorplatte 120 zu. In vorteilhafter Weise ist dadurch die Dämpfung der Übertragung des Schalls bzw. der Vibrationen auf den Sensor 12 sehr klein bzw. geht gegen null. Beispielsweise ist eine Verbindung des Sensors 12 auf der Sensorplatte 120 durch Kleben geeignet. Als Klebstoff kann ein ggf. Mehrkomponenten-Flüssigklebstoff oder alternativ ein Filmklebstoff eingesetzt werden. Ein Filmklebstoff kann z.B. ein Klebestreifen mit beidseitig aufgetragenem Klebefilm sein. Ferner kann die Verbindung Grundierungen oder dergleichen umfassen, mit denen die Sensorplatte 120 und/oder der Sensor 12 vor dem Klebevorgang versehen wird.

Gemäß einer weiteren Ausführungsform der Verbindung ist diese derart stoffschlüssig, dass der Sensor 12 mit der Sensorplatte eine unlösbare Einheit bildet. Eine derartige stoffschlüssige oder verpresste Einheit kann durch Vergießen erfolgen. Idealerweise ist der Sensor 12 in einer Ausnehmung der Sensorplatte 120 angeordnet und mit dieser mittels einer Vergussmasse vergossen. Alternativ kann der Sensor 12 auf der Oberfläche der Sensorplatte 120 mittels der beschriebenen Vergussmasse vergossen oder regelrecht aufgegossen sein.

Gemäß einer weiteren Ausführungsform der Verbindung ist diese mit einer Kraftbeaufschlagung versehen. Dabei wird mittels einer dauerhaft beaufschlagenden Kraft der Sensor 12 in Richtung auf die Sensorplatte 120 hin fixiert. Hierbei kann der Sensor 12 die Sensorplatte 120 direkt kontaktieren. Bevorzugt ist jedoch ein Sensorgehäuse vorgesehen, welches den Sensor 12 aufnimmt, sowie wenigstens dessen elektrischen Anschluss. Idealerweise ist das Sensorgehäuse mit der Sensorplatte 120 derart zusammenfügbar, so dass der Sensor 12 selbst nach Beendigung des Fügeprozesses eine feste Verbindung mit der Sensorplatte eingeht. Ferner können Kombinationen der eingangs genannten Verbindungen bestehen. Die Hinzunahme eines Sensorgehäuses ist prinzipiell von Vorteil, da es den Sensor 12 schützt und eine Kabelzugentlastung vornehmen kann. So kann alternativ ein Abdeckgehäuse einen fest mit der Sensorplatte 120 verklebter Sensor 12 schützend abdecken und eine Zugentlastung für das Anschlusskabel des Sensors 12 aufweisen. Das jeweilige Gehäuse ist ebenso fest beispielsweise durch Schrauben fest mit der Sensorplatte 120 verbunden.

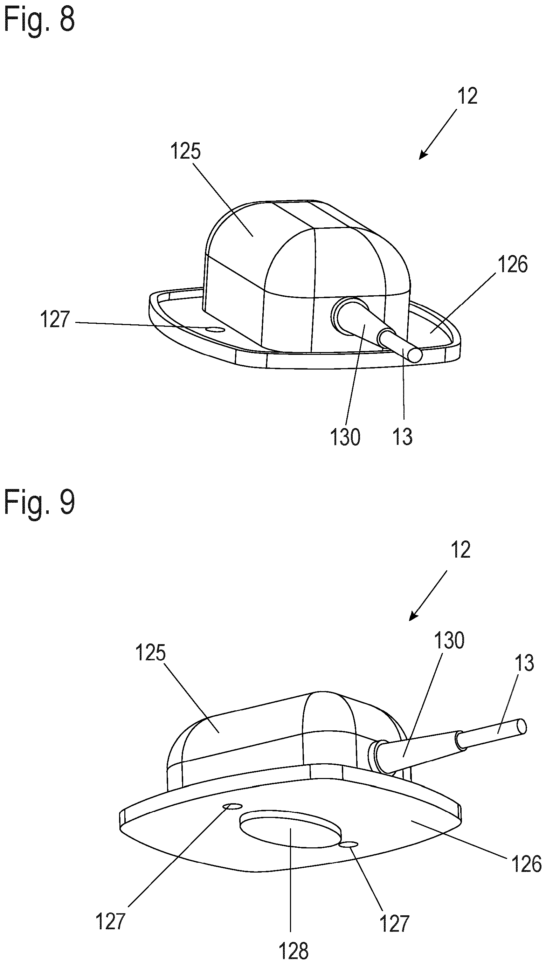

Ein Beispiel eines geeigneten Sensorgehäuses 125 eines Sensors ist in den Figuren 8 und 9 in jeweils einer isometrische Darstellung gezeigt. Dieser Sensor 12 kann beispielsweise bei den in den Figuren 3 bis 7 dargestellten Ausführungsbeispielen eingesetzt werden und ist auch beim Beispiel der Fig. 3 zu erkennen.

Das Sensorgehäuse 125 ist in der Fig. 8 in einer Schrägansicht auf seine Oberseite und in Fig. 9 in einer Schrägansicht mit Blick auf seine Unterseite wiedergegeben.

Das Sensorgehäuse 125 umfasst ein kuppelförmiges Gehäuseoberteil, in dem der eigentliche Schall- bzw. Schwingungsaufnehmer angeordnet ist. Eine Kabeldurchführung 130, vorzugsweise mit Knickschutz, dient der Durchführung des Sensorkabels 13. Das kuppelförmige Gehäuseteil ist mittig auf einer Montageplatte 126 angeordnet. Diese weist Befestigungslöcher 127 auf, beispielsweise für eine Schraubmontage.

Wie in Fig. 9 ersichtlich ist, ist an der Unterseite der Montageplatte 126, die dem Möbelteil, beispielsweise dem Rückenteil 4 zugewandt ist, eine Erhöhung 128 angeordnet, mit der der Sensor 12 bei Montage auf einer ebenen Fläche an dieser Fläche anliegt. Eine Schwingungsübertragung erfolgt damit insbesondere im Bereich der Erhöhung 128. Im Inneren des Sensorgehäuses 125 ist der eigentliche Schall- bzw. Schwingungsaufnehmer mit seinem schwingungsempfindlichen Bereich bevorzugt über der Erhöhung 128 angeordnet. Gegebenenfalls kann dazu die Erhöhung 128 nach innen fortgesetzt sein, sodass eine unmittelbare Übertragung auf den schwingungsempfindlichen Abschnitt des Schall- bzw. Schwingungsaufnehmers erfolgt. Auf diese Weise wird Körperschall möglichst unmittelbar auf den Schall- bzw. Schwingungsaufnehmer übertragen.

Im Gehäuse 121 ist zudem genügend Bauraum für Elektronik vorhanden, sodass eine Signalaufarbeitung des Sensorsignals unmittelbar im Sensorgehäuse 121 erfolgen kann. Diese Signalaufarbeitung kann eine Verstärkung und/oder Filterung des vom Schall- bzw. Schwingungsaufnehmer abgegebenen Signals beinhalten. Im Sensorkabel 13 können entsprechende Stromversorgungsleitungen für die Auswerteelektronik im Sensorgehäuse 121 vorgesehen sein. Neben einer Elektronik zur Signalvorverarbeitung kann auch die zuvor beschriebene Auswerteeinheit 9' unmittelbar im Sensorgehäuse 121 angeordnet werden.

Gemäß einer weiteren Ausführungsform ist der Sensor 12 während des Fertigungsprozesses der Sensorplatte 120 in diese eingebracht. Bei einer aus einem Kunststoff geformten Sensorplatte 120 ist der Sensor 12 beim Fertigungsprozess der Sensorplatte 120 in dieser eingegossen. Bei einer aus einem Faser-Holzwerkstoff geformten Sensorplatte 120 ist der Sensor 12 beim Fertigungsprozess der Sensorplatte 120 in dieser eingebracht. Bei einer aus einem Schicht-Holzwerkstoff geformten Sensorplatte 120 ist der Sensor 12 beim Fertigungsprozess der Sensorplatte 120 in dieser eingeklebt oder einlaminiert.

Alternative kann auch eine metallischen oder aus Kunststoff gefertigte Sensorplatte 120 eingesetzt werden.

### Bezugszeichenliste

- 1: Bett
- 2: Stützelement
- 3: Mittelteil
- 4: Rückenteil
- 5: Beinteil
- 6: Verbindung
- 60: Bewegungsbeschlag
- 7, 8: Verstellantrieb
- 9: Steuereinrichtung
- 10: Handbedienung
- 11: Übertragungsstrecke
- 12: Sensor
- 120: entkoppelter Abschnitt (Sensorplatte)
- 121: Einschnitt
- 125: Sensorgehäuse
- 126: Montageplatte
- 127: Befestigungsbohrung
- 128: Erhöhung
- 13: Sensorkabel
- 130: Knickschutz
- 14: Mobilgerät
- 15: drahtlose Signale

- 20: Signal
- 21: Herzschlag-Peak
- 22: Einhüllende
- 23: ansteigende Flanke (Einatmung)
- 24: abfallende Flanke (Ausatmung)

## Patentansprüche

1. Schlaf- oder Ruhemöbel mit einem Stützelement (2) zur Auflage eines Polsters oder Matratze und mindestens einem Sensor (12) zur Erfassung von Vibrationen, Bewegung und/oder Schall, wobei der Sensor (12) an einem Abschnitt (120) des Stützelements (2) angeordnet ist, der von weiteren Abschnitten des Stützelements (2) und/oder des Schlaf- oder Ruhemöbels akustisch entkoppelt ist, aufweisend eine mit dem Sensor (12) verbindbare Auswerteeinheit (9') die zur Verarbeitung und Auswertung der Signale des mindestens einen Sensors (12) und zur Erfassung von physiologischen Parametern einer das Schlaf- oder Ruhemöbel benutzenden Person eingerichtet ist, wobei das Schlaf- oder Ruhemöbel einen elektromotorischen Möbelantrieb mit Verstellantrieben (7, 8) zum Verstellen von Möbelteilen und einer Steuereinrichtung (9) zur Ansteuerung der Verstellantrieben (7, 8) aufweist, **dadurch gekennzeichnet, dass** die Auswerteeinheit (9') mit der Steuereinrichtung (9) gekoppelt ist oder in die Steuereinrichtung (9) integriert ist und dass der entkoppelte Abschnitt (120) durch mindestens einen Einschnitt (121, 122) von einem plattenförmigen Teil des Stützelements (2) abgeteilt ist, wobei mehrere voneinander durch verbleibende Stege getrennte Einschnitte (121) den entkoppelte Abschnitt (120) umgeben, und wobei der entkoppelte Abschnitt (120) durch die Stege mit dem umgebenden plattenförmigen Teil verbunden ist.

2. Schlaf- oder Ruhemöbel nach Anspruch 1, bei dem das plattenförmige Teil ein Mittelteil (3), ein Rückenteil (4) oder ein Beinteil (5) des Stützelements (2) ist.

3. Schlaf- oder Ruhemöbel nach Anspruch 1 oder 2, bei dem ein umlaufender Einschnitt (122) den entkoppelte Abschnitt (120) umgibt, wobei der entkoppelte Abschnitt (120) durch elastische Halteelemente (123) mit dem umgebenden plattenförmigen Teil verbunden ist.

4. Schlaf- oder Ruhemöbel nach einem der Ansprüche 1 bis 3, bei dem die erfassten physiologischen Parameter eine Herzfrequenz, eine Atemfrequenz, ein Bewegungszustand und/oder ein Schnarchzustand der Person sind.

5. Schlaf- oder Ruhemöbel nach einem der Ansprüche 1 bis 4, bei dem die Auswerteeinheit (9') einen Filter, insbesondere einen Tief- oder Bandpassfilter zur Signalverarbeitung aufweist.

6. Schlaf- oder Ruhemöbel nach einem der Ansprüche 1 bis 5, bei dem die Auswerteeinheit (9') der Signale des mindestens einen Sensors (12) zusätzlich zur Erfassung und Auswertung von Vibrationen eingerichtet ist, die bei der Betätigung eines oder mehrerer der Verstellantriebe (7, 8) auftreten.

7. Schlaf- oder Ruhemöbel nach Anspruch 6, bei dem die Auswerteeinheit (9') dazu eingerichtet ist, eine Fehlfunktion und/oder eine Überlastung und/oder eine Nicht-Belastung eines oder mehrerer der Verstellantriebe (7, 8) im Betrieb zu ermitteln.

## Claims

1. A piece of sleeping or reclining furniture, comprising a support element (2) for applying a padding or a mattress and at least one sensor (12) for detecting vibrations, movement, and/or sound, wherein
the sensor (12) is arranged at a section (120) of the support element (2) that is acoustically decoupled from further sections of the support element (2) and/or of the sleeping or reclining furniture, comprising an evaluation unit (9') connectable to the sensor (12), which is set up for processing and evaluating the signals of the at least one sensor (12) and for detecting physiological parameters of a person using the sleeping or reclining furniture, wherein the sleeping or reclining furniture comprises an electromotive furniture drive with adjusting drives (7, 8) for adjusting furniture parts and a control device (9) for controlling the adjusting drives (7, 8), **characterized in, that** the evaluation unit (9') is coupled to the control device (9) or is integrated into the control device (9) and that the decoupled section (120) is divided by at least one cut (121, 122) from a plate-shaped part of the support element (2), wherein a plurality of cuts (121) separated from each other by remaining webs surround the decoupled section (120), and wherein the decoupled section (120) is connected with the surrounding plate-shaped part by the webs.

2. The piece of sleeping or reclining furniture of claim 1, wherein the decoupled section (120) is a middle part (3), a back part (4) or a leg part (5) of the support element (2).

3. The piece of sleeping or reclining furniture of claim 1 or 2, wherein a circumferential cut (122) surrounds the decoupled section (120), and wherein the decoupled section (120) is connected by elastic holding elements (123) with the surrounding plate-shaped part.

4. The piece of sleeping or reclining furniture of one of claims 1 to 3, wherein the detected physiological parameters are a heart rate, a respiratory rate, a movement behavior and/or a snoring behavior of the person.

5. The piece of sleeping or reclining furniture of one of claims 1 to 4, wherein the evaluation unit (9') comprises a filter, in particular a low-pass or bandpass filter for signal processing.

6. The piece of sleeping or reclining furniture of one of claims 1 to 5, wherein the evaluation unit (9') for the signals of the at least one sensor (12) is additionally set up for the detection and evaluation of vibrations and movement that occur when one or more of the adjustment drives (7, 8) are actuated.

7. The piece of sleeping or reclining furniture of claim 6, wherein the evaluation unit (9') is designed to determine a malfunction and/or an overload and/or a non-load of one or more of the adjusting drives (7, 8) during operation.

## Revendications

1. Meuble de couchage ou de repos avec un élément d'appui (2) pour poser un rembourrage ou un matelas et avec au moins un capteur (12) pour détecter des vibrations, des mouvements et/ou des sons, dans lequel le capteur (12) est disposé sur une section (120) de l'élément d'appui (2) qui est découplée acoustiquement d'autres parties de l'élément d'appui (2) et/ou du meuble de couchage ou de repos, comportant une unité d'analyse (9') qui peut être reliée au capteur (12) et qui est conçue pour traiter et analyser les signaux de l'au moins un capteur (12) et pour détecter des paramètres physiologiques d'une personne utilisant le meuble de couchage ou de repos, lequel meuble de couchage ou de repos comporte un entraînement par moteur électrique avec des entraînements de manœuvre (7, 8) pour manœuvrer des parties de meuble et un dispositif de commande (9) pour actionner les entraînements de manœuvre (7, 8), **caractérisé en ce que** l'unité d'analyse (9') est couplée au dispositif de commande (9) ou intégrée dans le dispositif de commande (9) et **en ce que** la section découplée (120) est séparée par au moins une encoche (121, 122) d'une partie en forme de plaque de l'élément d'appui (2), la section découplée (120) étant entourée par plusieurs encoches (121) reliées par des barrettes restantes et la section découplée (120) étant reliée par les barrettes à la partie en forme de plaque environnante.

2. Meuble de couchage ou de repos selon la revendication 1, dans lequel la partie en forme de plaque présente une partie médiane (3), une partie pour le dos (4) ou une partie pour les jambes (5) de l'élément d'appui (2).

3. Meuble de couchage ou de repos selon la revendication 1 ou 2, dans lequel une encoche périphérique (122) entoure la section découplée (120), laquelle section découplée (120) est reliée à la partie en forme de plaque environnante par des éléments de rétention élastiques (123).

4. Meuble de couchage ou de repos selon l'une des revendications 1 à 3, dans lequel les paramètres physiologiques captés sont une fréquence cardiaque, une fréquence respiratoire, un état de mouvement et/ou un état de ronflement de la personne.

5. Meuble de couchage ou de repos selon l'une des revendications 1 à 4, dans lequel l'unité d'analyse (9') comporte un filtre, en particulier un filtre passe-bas ou un filtre passe-bande pour le traitement des signaux.

6. Meuble de couchage ou de repos selon l'une des revendications 1 à 5, dans lequel l'unité d'analyse (9') des signaux de l'au moins un capteur (12) est en outre conçue pour capter et analyser des vibrations qui se produisent lors de l'actionnement d'un ou plusieurs des entraînements de manœuvre (7, 8).

7. Meuble de couchage ou de repos selon la revendication 6, dans lequel l'unité d'analyse (9') est conçue pour déterminer un dysfonctionnement et/ou une surcharge et/ou une absence de charge d'un ou plusieurs des entraînements de manœuvre (7, 8).
